# EUROPÄISCHE PATENTSCHRIFT

(11) **EP 2 567 717 B1**
(45) Veröffentlichungstag und Bekanntmachung des Hinweises auf die Patenterteilung: **26.11.2014**
(21) Anmeldenummer: 12006176.7
(22) Anmeldetag: 31.08.2012
(51) Int. Cl.: A61M 1/00, G09B 23/28, G09B 23/30

(54) **Vorrichtung zur Simulation einer Wunde am offenen Abdomen**
Device for simulating a wound on the open abdomen
Dispositif destiné à simuler une plaie sur un abdomen ouvert

(30) Priorität: 09.09.2011 DE 102011112795
(43) Veröffentlichungstag der Anmeldung: 13.03.2013
(73) Patentinhaber: Paul Hartmann AG, 89522 Heidenheim (DE)
(72) Erfinder: Croizat, Pierre, 89542 Herbrechtingen (DE); Junginger, Martin, 89568 Hermaringen (DE); Wolf, Cornelia, 89542 Bohlheim (DE)

(56) Entgegenhaltungen:
- WO-A1-2010/072349
- GB-A- 2 416 909
- US-A1- 2008 077 091
- US-A1- 2009 098 521
- US-A1- 2010 268 197

## Beschreibung

Die Erfindung betrifft eine Vorrichtung zur Simulation einer Wunde am Körper eines Säugetiers (Wundsimulator), insbesondere zur Simulation einer Wunde am offenen Abdomen. Die Erfindung betrifft weiterhin eine Messvorrichtung zum Testen von Verbänden, welche für die Unterdrucktherapie von Wunden, insbesondere zur Unterdrucktherapie von Wunden im Abdominalbereich, vorgesehen sind.

Vorrichtungen und Verbände zur Unterdrucktherapie von Wunden sind im Stand der Technik bekannt. Die Unterdrucktherapie von Wunden wird häufig mit dem Ausdruck "NPWT" (engl.: negative pressure wound therapy) bezeichnet.

So beschreibt beispielsweise die WO1993/009727 eine Vorrichtung zur Förderung der Wundheilung durch die Applikation von Unterdruck auf dem die Wunde aufweisenden und die Wunde umgebenden Hautbereich. Die Vorrichtung gemäß WO1993/009727 umfasst eine Vakuumeinrichtung zur Erzeugung des Unterdrucks, eine als Dichtungseinrichtung bezeichnete luftdichte Abdeckung der Wunde, welche mit der Vakuumeinrichtung in einer funktionellen Verbindung steht, sowie eine als Schutzeinrichtung bezeichnete Wundauflage zur Positionierung an der Wunde innerhalb der Dichtungseinrichtung. Bei der Schutzeinrichtung handelt es sich um einen porösen Polymerschaumstoff, beispielsweise um Polyester-Schaum. Ausweislich der Beschreibung von WO1993/009727 kann durch die Anwendung der Unterdrucktherapie die Wundheilung unterschiedlich Typen von Wunden wie beispielsweise Brandwunden, Druckwunden oder Platzwunden beschleunigt werden.

Besonders großflächige Wunden können im Abdominalbereich entweder infolge von Verletzungen oder infolge von chirurgischen Eingriffen entstehen. Chirurgische Eingriffe im Abdominalbereich werden beispielsweise bei der operativen Behandlung akuter und lebensbedrohlicher Erkrankungen des Bauchraums vorgenommen. Im Rahmen der postoperativen Versorgung derartiger chirurgischer Eingriffe kann auch die Notwendigkeit bestehen, den offenen Abdominalbereich mittels eines temporären Wundverschlusses nur vorübergehend abzudecken.

Bei der Behandlung offener Wunden im Abdominalbereich mittels Unterdrucktherapie kann es erforderlich sein, sehr große Mengen an Wundsekret, bis zu 4 I pro Tag, aus dem Wundraum abzuführen. Bei der Entwicklung moderner Wundverbände zur Unterdrucktherapie des offenen Abdomens stellt sich daher die Herausforderung eine ausreichend starke Drainagekapazität zu gewährleisten. Gleichzeitig müssen zur Herstellung der Wundverbände Materialien verwendet werden, welche für den Patienten verträglich sind.

Bei der Auswahl geeigneter Materialien kann der Fachmann auf die Erfahrung in der Wundtherapie allgemein und insbesondere auf die Erfahrung im Bereich der Unterdrucktherapie zurückgreifen. Ggf. muss die Verträglichkeit der verwendeten Verbandmaterialien zunächst in Tierversuchen und dann in klinischen Versuchen überprüft.

Zur Überprüfung der Drainagekapazität von Wundverbänden für die Unterdrucktherapie kann als Testsystem eine als "künstliche Wunde" bezeichnete Vorrichtung verwendet werden. Ein auf einer künstlichen Wunde basierendes Testsystem ermöglicht eine Messung der Drainagekapazität des Unterduckverbands unter standardisierten Bedingungen. Eine "künstliche Wunde" ist dem Fachmann unter der Bezeichnung Wundsimulator bekannt. Wundsimulatoren werden sowohl zur Verbesserung bekannter Wundverbände, als auch zur Entwicklung neuartiger Wundverbände eingesetzt. Durch den Einsatz einer Wundsimulations-Vorrichtung können zumindest diejenigen Tierversuche vermieden werden, deren Untersuchungsgegenstand die Drainagekapazität des Wundverbandes ist.

Darüber hinaus ermöglichen Wundsimulatoren einem Anwender, beispielsweise einem Chirurgen, sich vor der Applikation eines Verbandes am Patienten mit dem Therapiesystem vertraut zu machen. Derartige Wundsimulatoren sind gleichfalls als Lehr- und Demonstrationsobjekte für die medizinische Ausbildung geeignet.

Eine System zum Testen von Unterdruckwundaufklagen ist aus der US 2008/0077091 bekannt. Hierzu ist eine simulierte Wunde mit einem Gehäuse und einer Wundkavität innerhalb des Gehäuses vorgesehen. Über die Form und die Größe der Kavität wird ein bestimmter Wundtyp repräsentiert. Über dieser Wundkavität kann ein Wundverband positioniert werden und es wird eine Unterdruckeinrichtung an die Wundkavität angelegt.

Des Weiteren wird die Vorsehung einer Fluidquelle zur Bereitstellung von Fluiden in der Wundkavität, die der Simulation von Wundexsudat dienen soll, offenbart.

Des Weiteren ist aus der Dissertation "Druckverhältnisse im Bereich der Grenzschicht Schwamm/Gewebe während der Anwendung der Vakuumversiegelung" von Hinnerk von Thun-Hohenstein, Osnabrück, 2006 ein Testsystem bekannt, das der Überprüfung von Wundauflagen unter Unterdruckbedingungen dient, wobei die Messanordnung die Verwendung eines Rinderherzens oder einer realen Wunde bedarf.

Eine weitere Testapparatur ist aus der WO-2010/072349 bekannt, die eine Messapparatur für Unterdrucktherapiesysteme beschreibt. Die Vorrichtung umfasst eine Wundeinheit mit einer künstlichen Wundkavität, wobei in der Wandung der Kavität Fluiddurchlässe vorgesehen sind, so dass über Zuleitungen ein Fluid in den künstlichen Wundraum eingebracht werden kann. Die offene Seite der Wundkavität kann mit einem Unterdrucktherapieverband abgedeckt werden. Weiterhin sieht die WO-2010/072349 eine regelbare Heizeinrichtung zur Temperierung der Wundeinheit und/oder des Fluids vor.

Nachteilig bei den genannten Messapparaturen für Unterdrucktherapiesysteme ist dabei, dass die Oberflächenstruktur der Wunde nur mangelhaft abgebildet werden kann.

Insbesondere ist aus dem Stand der Technik keine Vorrichtung bekannt, welches die speziellen Gegebenheiten und die Oberflächenstruktur einer Wunde am offenen Abdomen unter realistischen Einsatzbedingungen abbilden könnte.

Ein Wundsimulator zur Testung eines Unterdrucktherapieverbandes, welcher zur Unterdruck-Behandlung des offenen Abdomens vorgesehen ist (im Folgenden auch als "Abdominalverband" oder "NPWT-Abdominalverband" bezeichnet), steht derzeit nicht zur Verfügung.

Es ist Aufgabe der Erfindung, den Stand der Technik weiter zu verbessern. Insbesondere ist es Aufgabe der Erfindung eine Vorrichtung für die Testung, Verbesserung und Neuentwicklung von Unterdrucktherapieverbänden, welche zur Behandlung des offenen Abdomens vorgesehen sind bereitzustellen, wobei die natürliche Wundumgebung möglichst gut abgebildet werden soll. Eine derartige Vorrichtung soll darüber hinaus mit einem vertretbaren Aufwand hergestellt werden können, so dass die Vorrichtung auch als Lehr-, Demonstrations- und Übungsobjekt, beispielsweise für Medizinstudenten oder Chirurgen, eingesetzt werden kann.

Die Erfindung löst diese Aufgabe durch eine Vorrichtung zur Simulation einer Wunde am Körper eines Säugetiers, insbesondere zur Simulation einer Wunde am offenen Abdomen, mit den Merkmalen des Anspruchs 1. Erfindungsgemäß ist mindestens ein mit einem ersten Fluid gefüllter erster Hohlkörper mit flexibler Wandstruktur, sowie ein zweiten Hohlkörper mit einer flexiblen äußeren Hüllstruktur vorgesehen, wobei die äußere Hüllstruktur des zweiten Hohlkörpers mindestens eine erste Öffnung aufweist, welche eine Wunde simulieren kann. Der mindestens eine erste Hohlkörper muss dabei in das Innere des zweiten Hohlkörpers einbringbar sein, d.h. erste und zweite Hohlkörper müssen hinsichtlich ihrer Größe derart dimensioniert sein, dass der mindestens eine erste Hohlkörper vollständig in den Hohlraum des zweiten Hohlkörpers eingebracht werden kann. Erfindungsgemäß sind ein oder vorzugsweise mehrere erste Hohlkörper, jedoch nur ein einziger zweiter Hohlkörper vorgehen. Falls mehr als ein erster Hohlkörper vorgesehen ist, so müssen alle ersten Hohlkörper in ihrer Gesamtheit in den Hohlraum des zweiten Hohlkörpers einbringbar sein.

Der zweite Hohlkörper weist eine erste Öffnung auf, welche eine Wunde simulieren kann. Die erste Öffnung sollte beim Einsatz der Vorrichtung möglichst nach oben orientiert sein. Die erste Öffnung sollte hinsichtlich ihrer Abmessungen der zu simulierenden Wunde entsprechen.

Der eine oder die mehreren ersten Hohlkörper sollen den Innenraum des zweiten Hohlkörpers weitestgehend ausfüllen, so dass sich die Oberfläche des einen oder der mehreren ersten Hohlkörper in der Nähe, jedoch unterhalb der Ebene der ersten Öffnung befindet.

Die erfindungsgemäße Vorrichtung wird im Folgenden auch als Wundsimulator bzw. als NPWT-Wundsimulator bezeichnet. Der mit der vorliegenden Erfindung beschriebene Wundsimulator ist insbesondere zur Testung von Wundverbänden für die Unterdrucktherapie vorgesehen. Er eignet sich im besonderen Maße für die Testung von NPWT-Abdominalverbänden.

Der erfindungsgemäße Wundsimulator kann gleichfalls in vorteilhafter Weise zur Testung von Verbänden eingesetzt werden, die für eine so genannte "Instill-Therapie" vorgesehen sind. Bei der Instill-Therapie handelt es sich um eine Variante der Unterdrucktherapie von Wunden, bei der eine Flüssigkeit, beispielsweise eine Spüllösung oder eine die Wundheilung in anderer Weise fördernde Flüssigkeit, in den Wundraum eingebracht wird. Eine "Instill-Vorrichtung " ist beispielsweise in der WO2005/102415 beschrieben.

Es ist darüber hinaus vorstellbar, dass der Wundsimulator auch für die Testung von Wundverbänden eingesetzt werden kann, die nicht für die Unterdrucktherapie und/oder für die Instill-Therapie vorgesehen sind, beispielsweise zur Testung absorbierender Wundauflagen.

Der mit der vorliegenden Erfindung beschriebene Wundsimulator ermöglicht die Simulation einer menschlichen oder tierischen Wunde. Im Simulations-Experiment wird ein als künstliches Wundexsudat dienendes zweites Fluid über die erste Öffnung in das Innere des zweiten Hohlkörpers eingebracht. Der zu testende Verband wird im Bereich der ersten Öffnung des zweiten Hohlkörpers appliziert, wobei die Wundauflage gleichzeitig in Kontakt mit der Oberfläche des oder der ersten Hohlkörper gebracht wird. Die Oberfläche des oder der ersten Hohlkörper simuliert dabei den Wundgrund, während der Rand der ersten Öffnung dem Wundrand einer realen Wunde am menschlichen oder tierischen Körper entspricht. Die Wundauflage bzw. der Verband kann in dem die erste Öffnung umgebenden Bereich auf der äußeren Oberfläche des zweiten Hohlkörpers fixiert werden. Dieser Bereich entspricht folglich der die Wunde umgebenden intakten Haut bei einer realen Wunde am menschlichen oder tierischen Körper.

Die Form der künstlichen Wunde (erste Öffnung im zweiten Hohlkörper) kann der Zielsetzung des Simulationsexperimentes angepasst werden. Die erste Öffnung im zweiten Hohlkörper kann dabei jede gewünschte Größe und Form aufweisen, beispielsweise rund, oval oder eine unregelmäßige Form. Es ist jedoch darauf zu achten, dass die offene Fläche der ersten Öffnung im zweiten Hohlkörper einen Wert von etwa 35 % der Gesamtoberfläche (offene und geschlossene Fläche) des zweiten Hohlkörper nicht überschreitet, da ansonsten die Stabilität der Vorrichtung in unerwünschter Weise reduziert wird.

Der mindestens eine mit einem ersten Fluid gefüllte erste Hohlkörper simuliert dabei aufgrund seiner Oberflächenbeschaffenheit in vorteilhafter Weise die Gegebenheiten bei einer realen Wunde, insbesondere bei einer Wunde im Abdominalbereich. Dies trifft insbesondere dann zu, wenn eine Vielzahl von mit einem ersten Fluid gefüllten ersten Hohlkörpern verwendet wird.

Während des Simulations-Experimentes kann das im Inneren des zweiten Hohlkörpers vorhandene künstliche Wundexsudat über den zu testenden Wundverband abgesaugt werden.

Generell können der erste Hohlkörper und / oder der zweite Hohlkörper beispielsweise aus einem Kunststoff, aus einem gummiartigen Material oder aus Kautschuk gefertigt sein.

Vorzugsweise umfasst der erste Hohlkörper und / oder der zweite Hohlkörper einen elastischen Kunststoff, Gummi, Naturkautschuk oder eine Mischung daraus.

Hinsichtlich der Handhabung des Wundsimulators hat es sich weiterhin als besonders praktisch erwiesen, wenn der zweite Hohlkörper eine transparente Hüllstruktur aufweist. Eine transparente Hüllstruktur ermöglicht eine Beobachtung des künstlichen Wundexsudates (zweites Fluid) während des Simulationsexperimentes, insbesondere wenn das künstliche Wundexsudat angefärbt ist. Bei dem zweiten Fluid kann es sich beispielsweise um eine rot angefärbte Blutersatzlösung handeln.

Gemäß einer besonders einfach und kostengünstig herstellbaren Ausführungsform, die beispielsweise für Ausbildungs- oder Demonstrationszwecke geeignet ist, kann eine erfindungsgemäße Vorrichtung aus einem Gymnastikball und einem oder mehreren handelsüblichen Luftballons gefertigt werden. Um einen derartigen Wundsimulator herzustellen, wird in den Gymnastikball eine Öffnung geschnitten, welche als künstliche Wunde dient. Durch diese Öffnung werden vorzugsweise mehrere mit einer Flüssigkeit gefüllte Luftballons in das innere des Gymnastikballs eingebracht, bis der innere Hohlraum des Gymnastikballs weitestgehend mit den Luftballons aufgefüllt ist.

Der zweite Hohlkörper sollte ein Volumen von mindestens 1.500 cm³ und höchstens 600.000 cm³ aufweisen, vorzugsweise mindestens 5.000 cm³ und höchstens 400.000 cm³. Unter Volumen eines Hohlkörpers wird hierbei und im Folgenden das Volumen eines Hohlkörpers im ungedehneten Zustand verstanden. Ein zweiter Hohlkörper mit einem geringeren Volumen als 1.500 cm³ kann aufgrund seiner geringen Oberfläche nicht mehr zur Testung üblicher Wundauflagen eingesetzt werden. Ein zweiter Hohlkörper mit einem höheren Volumen als 600.000 cm³ ist zu schwer und unhandlich.

Bei dem zur Füllung des ersten Hohlkörpers verwendbaren Fluid kann es sich um ein beliebiges Gas, beispielsweise um Luft, Kohlendioxid oder Stickstoff, oder um eine beliebige Flüssigkeit handeln. Bei Verwendung eines Gases zur Füllung der ersten Hohlkörper bleibt die Vorrichtung insgesamt wesentlich leichter und kann daher einfacher transportiert werden. Vorzugsweise wird zur Füllung des oder der ersten Hohlkörper jedoch eine Flüssigkeit, insbesondere Wasser, eingesetzt, da bei Verwendung einer Flüssigkeit eine hinsichtlich ihrer elastischen Eigenschaften den natürlichen Gegebenheiten eines Wundgrundes ähnliche Oberflächenbeschaffenheit gebildet wird. Alternativ können auch Flüssigkeiten mit gegenüber Wasser veränderter Viskosität verwendet werden, beispielsweise Glycerin. Der Fachmann kann durch eine Auswahl geeigneter Flüssigkeiten die Oberflächenbeschaffenheit der ersten Hohlkörper in gewünschter Weise einstellen.

Bei dem zweiten Fluid handelt es sich vorzugsweise um eine Flüssigkeit. Bei der Auswahl derartiger künstlicher Wundflüssigkeiten kann sich der Fachmann ohne Einschränkungen an der Zielsetzung des Versuchs orientieren. Im einfachsten Fall wird Wasser eingesetzt, das zusätzlich angefärbt werden kann. Vorteilhafterweise werden jedoch an sich aus dem Stand der Technik bekannte künstliche Wundexsudate eingesetzt, welche hinsichtlich ihrer Konsistenz die Gegebenheiten einer realen Wunde abbilden. Beispiele hierfür sind wässrige Lösungen mit einem Farbstoff, Glycerin und Natriumchlorid (z.B. 566 g demineralisiertes Wasser, 425 g Glycerin, 9 g NaCl, 0,2 g Allura Red), wässrige Lösungen mit einem Farbstoff und Xanthan (z.B. 1000 g demineralisiertes Wasser, 2 g Xanthan, 0,2 g Allura Red), sowie ein künstliches Wundexsudat, welches aus einer Vollei-Lösung besteht.

Das künstliche Wundexsudat kann vor dem Simulations-Experiment durch die erste Öffnung in das Innere des zweiten Hohlkörpers eingebracht werden.

Gemäß einer sehr vorteilhaften Weiterentwicklung der Erfindung ist jedoch eine kontinuierliche Zufuhr des künstlichen Wundexsudates vorgesehen, welche über mindestens eine zweite Öffnung am zweiten Hohlkörper erfolgen kann. Gemäß dieser Ausführungsform weist die Vorrichtung deshalb einen ersten Hohlkörper mit mindestens einer zweiten Öffnung auf, welche die Zufuhr, insbesondere eine kontinuierliche Zufuhr, des zweiten Fluids in das Innere des zweiten Hohlkörpers ermöglicht.

Im Rahmen besonderer Experimente könnte über die zweite Öffnung anstelle von Flüssigkeit oder alternierend zur Applikation von Flüssigkeit auch ein Gas als zweites Fluid eingebracht werden. Dies kann beispielsweise zur Simulation spezieller Wunden im Darmbereich erforderlich sein.

Grundsätzlich ist es möglich den erfindungsgemäßen Wundsimulator aus nur einem einzigen mit einem ersten Fluid gefüllten Hohlkörper, welcher sich im Inneren des zweiten Hohlkörpers befindet, zu konstruieren. Nach dem Befüllen des Wundsimulators befindet sich das künstliche Wundexsudat zwischen dem ersten und dem zweiten Hohlkörper. Die zu testende Wundauflage liegt dann mit ihrer Unterseite auf der Oberfläche des ersten Hohlkörpers. Entsprechend wird das künstliche Wundexsudat durch die zu testende Wundauflage im Randbereich der Wundauflage aufgenommen, während eine Absaugung von Exsudat über die Fläche der Wundauflage nicht oder nur im geringen Maße stattfindet. Ein derartiger Aufbau des Wundsimulators kann beispielsweise dann vorteilhaft sein, wenn die Absorptions- bzw. Drainagekapazität der Wundauflage im Randbereich untersucht werden soll.

Sollte es dagegen erwünscht sein, dass eine Absaugung des künstlichen Wundexsudates gleichzeitig über die Fläche der Wundauflage erfolgt, so sollte die Vorrichtung mindestens 3 und höchstens 1.000 der ersten Hohlkörper, vorzugsweise mindestens 25 und höchstens 150 der ersten Hohlkörper, besonders bevorzugt mindestens 50 und höchstens 100 der ersten Hohlkörper, welche in das Innere des zweiten Hohlkörpers einbringbar sind, umfassen. Bei der Verwendung einer Vielzahl von ersten Hohlkörpern ergibt sich eine Vielzahl von Zwischenräumen, in denen sich das zweite Fluid befinden kann. Da die zu testende Wundauflage im Bereich des künstlichen Wundgrundes der künstlichen Wunde (erste Öffnung im zweiten Hohlkörper) mit diesen Zwischenräumen in Kontakt tritt, wird eine Absaugung des künstlichen Wundexsudates über die Fläche der Wundauflage ermöglicht. Des Weiteren ergibt bei der Verwendung einer Vielzahl von ersten Hohlkörpern im Bereich des Wundgrundes der künstlichen Wunde eine Oberflächenbeschaffenheit, die insbesondere dem Wundgrund einer realen abdominalen Wunde sehr nahe kommt.

Die Größe und Anzahl des oder der ersten Hohlkörpers kann den gewünschten Versuchsbedingungen angepasst werden. Insbesondere kann durch Größe und Anzahl des oder der ersten Hohlkörpers die Oberflächenbeschaffenheit des künstlichen Wundgrundes eingestellt werden. Bei der Verwendung nur eines einzigen ersten Hohlkörpers sollte dessen Volumen im befüllten Zustand geringfügig (beispielsweise 1 % bis 5 %) unter dem Volumen des zweiten Hohlkörpers liegen. Bei der Verwendung mehrerer erster Hohlkörper müssen diese naturgemäß kleiner ausgewählt werden. In jedem Falle sollten die ersten Hohlkörper ein Volumen von mindestens 0,5 cm³ und weniger als 300.000 cm³ aufweisen, falls mehr als ein erster Hohlkörper verwendet wird.

Bei der Verwendung einer Vielzahl von ersten Hohlkörpern kann es erwünscht sein die mit einem ersten Fluid gefüllten ersten Hohlkörper in Ihrer Gesamtheit in eine zusätzliche sackartige Umhüllung einzubringen, wobei die zusätzliche Umhüllung vorzugsweise aus einem flexiblen Folienmaterial mit einer Dicke von weniger als 2 mm besteht. Eine derartige zusätzliche Umhüllung ersten Hohlkörper kann die Stabilität der Vorrichtung verbessern und ermöglicht eine Modifikation der Oberflächenbeschaffenheit des künstlichen Wundgrundes der künstlichen Wunde (erste Öffnung im zweiten Hohlkörper).

Insbesondere zur Simulation von Abdominalwunden kann es vorteilhaft sein, zumindest die Umgebung der künstlichen Wunde (erste Öffnung im zweiten Hohlkörper) mit einer zusätzlichen Lage eines flexiblen Materials auszustatten, welche auf der Innenseite der Hüllstruktur des zweiten Hohlkörpers aufgebracht ist. Eine weitere vorteilhafte Ausführungsform der Erfindung betrifft deshalb eine erfindungsgemäße Vorrichtung wie vorstehend dargestellt, wobei die Vorrichtung weiterhin eine zusätzliche Lage eines flexiblen Materials zur Auskleidung zumindest eines Anteils der inneren Seite der Hüllstruktur des zweiten Hohlkörpers umfasst, und wobei die zusätzliche Lage eine Dicke von mindestens 0,5 cm und höchstens 5,0 cm aufweist.

Vorzugsweise ist mindestens 10 %, insbesondere mindestens 50 % der inneren Seite der Hüllstruktur des zweiten Hohlkörpers mit der zusätzlichen Lage eines flexiblen Materials ausgekleidet. Alternativ kann auch die gesamte Oberfläche der inneren Seite der Hüllstruktur des zweiten Hohlkörpers mit der zusätzlichen Lage eines flexiblen Materials ausgekleidet sein. Klarstellend wird angemerkt, dass die zusätzlichen Lage eines flexiblen Materials innerhalb des Umfangs der künstlichen Wunde (erste Öffnung im zweiten Hohlkörper) nicht vorhanden ist, d.h. die erste Öffnung geht sowohl durch die Hüllstruktur des zweiten Hohlkörpers, als auch durch die zusätzlichen Lage eines flexiblen Materials, falls vorhanden.

Eine derartige zusätzliche Lage eines flexiblen Materials kann die Stabilität der Vorrichtung verbessern.

Weiterhin ermöglicht die zusätzliche Lage, insofern diese in der unmittelbaren Umgebung der der künstlichen Wunde (erste Öffnung im zweiten Hohlkörper) vorhanden ist, eine Adaption der Dicke des künstlichen Wundrandes an die bei einer realen Wunde vorhandenen Gegebenheiten, beispielsweise an die Dicke einer menschlichen Bauchdecke. Die Dicke (Abmessung senkrecht zur Flächenerstreckung) des künstlichen Wundrandes ergibt sich bei der Verwendung einer zusätzlichen Lage eines flexiblen Materials aus der Summe der Dickenerstreckung der Hüllstruktur des zweiten Hohlkörpers und der Dickenerstreckung der zusätzlichen Lage.

Die zusätzliche Lage des flexiblen Materials kann mit der inneren Seite der Hüllstruktur des zweiten Hohlkörpers entweder unlösbar verbunden ist oder lediglich lose eingelegt werden. Eine unlösbare Verbindung ist beispielsweise durch Verkleben möglich. Ziel der Anwender darauf ab, durch den Einsatz einer zusätzlichen Lage des flexiblen Materials die Gegebenheiten einer menschlichen Bauchdecke zu simulieren, so sollte die zusätzliche Lage zumindest in der Umgebung der künstlichen Wunde fest verbunden werden, beispielsweise durch Ankleben der zusätzlichen Lage an die inneren Seite der Hüllstruktur des zweiten Hohlkörpers.

Bei der zusätzlichen Lage des die innere Seite der Hüllstruktur des zweiten Hohlkörpers zumindest teilweise auskleidenden Materials kann es sich insbesondere um eine Lage aus einem geschäumten oder ungeschäumten Kunststoff, beispielsweise um Polyurethan, Polyethylen, Polypropylen, Polyvinylchlorid, Polyethylenterephthalat oder eine Mischung daraus handeln. Eine aus einem geschäumten elastomeren Kunststoff gefertigte Lage ist bevorzugt.

Durch die Verwendung einer regelbaren Heiz- und/oder Kühleinrichtung kann die zu testende Wundauflage über die Temperaturregelung der künstlichen Wunde, sowie über die Temperaturregelung der künstlichen Wunde zugeführte Wundfluid realitätsnäher getestet werden, da sich eine beheizte Wunde und ein vorgewärmtes künstliches Wundexsudat näher an den tatsächlichen Gegebenheiten einer Wunde befinden als eine Durchführung von Tests bei Raumtemperatur. Insbesondere lassen sich realitätsnahe Wundsituationen bei einer der Körpertemperatur angenäherten Temperatur durchführen. Des Weiteren gewährleistet eine derartige Heiz- und/oder Kühleinrichtung unabhängig von den Umgebungstemperaturen die Durchführung des Versuchs bei konstanten Temperaturbedingungen. Besonders bevorzugt ist deshalb ein regelbares Heiz- bzw. Wasserbad zur Vortemperierung der Fluids, das als künstliches Wundexsudat dient.

Gemäß einer weiteren bevorzugten Ausführung der Erfindung weist der zweite Hohlkörper deshalb mindestens eine vierte Öffnung auf, welche zum Anschluss eines Heiz- und/oder eines Kühlelementes vorgesehen ist, so dass die im Inneren des zweiten Hohlkörpers vorhandene Temperatur eingestellt werden kann. Bei dem Heiz- und/oder eines Kühlelemente kann es sich beispielsweise um geschlossene Wasserleitung handeln, welche mit einem mit einer Temperaturregelung ausgestattetem Wasserbad verbunden ist. Ein Anteil der Wasserleitung wird dabei durch das Innere des zweiten Hohlkörpers gelegt. Das durch die Leitung strömende Wasser bewirkt einen Angleich der Temperatur im Inneren des zweiten Hohlkörpers an die Temperatur des durchströmenden Wassers. Um eine gewünschte Temperatureinstellung im Inneren des zweiten Hohlkörpers zu erreichen, sollte mit der Temperierung - je nach Differenz zwischen der vor der Temperierung im Inneren des zweiten Hohlkörpers vorhandenen Temperatur und der zu erreichenden Temperatur - bereits mehrere Stunden vor dem Test der Wundauflage begonnen werden.

Idealerweise sollte der Wundsimulator über beide der vorgenannten Temperierungseinrichtungen verfügen, d.h. es sollte sowohl eine Temperatureinstellung des zugeführten Fluids ermöglicht werden, als auch eine Temperierungseinrichtung im Inneren des zweiten Hohlkörpers vorgesehen sein. Technisch ist es dabei vorteilhaft beide Temperierungseinrichtungen über ein einziges Wasserbad zu realisieren.

Weiterhin ist es vorteilhaft, wenn die Vorrichtung mindestens eine dritte Öffnung aufweist, welche zum Anschluss eines Drucksensors und/oder eines Temperatursensors vorgesehen ist, so dass der im Inneren des zweiten Hohlkörpers vorhandene Innendruck Pᵢ und/oder die im Inneren des zweiten Hohlkörpers vorhandene Temperatur Tᵢ bestimmt werden kann. Die Reproduzierbarkeit des Simulationsexperimentes kann durch die Anwendung derartiger Sensoren verbessert werden. Die Sensordaten sollten möglichst über die gesamte Zeit des Experiments hinweg elektronisch aufgezeichnet werden.

Die vorliegende Erfindung umfasst eine Messvorrichtung für Wundverbände, insbesondere für Wundverbände, welche zur Unterdrucktherapie von Wunden am offenen Abdomen vorgesehen sind, eingesetzt werden. Eine Messvorrichtung für Wundverbände umfasst
(i) mindestens einen mit einem ersten Fluid gefüllten ersten Hohlkörper mit flexibler Wandstruktur,
(ii) einen zweiten Hohlkörper mit einer flexiblen äußeren Hüllstruktur, wobei die äußere Hüllstruktur mindestens eine erste Öffnung aufweist, welche eine Wunde simulieren kann, wobei weiterhin der mindestens ein erster Hohlkörper in das Innere des zweiten Hohlkörpers einbringbar ist,
(iii) optional eine zusätzliche Lage eines flexiblen Materials, welches die innere Seite der Hüllstruktur des zweiten Hohlkörpers zumindest teilweise auskleidet, wobei die zusätzliche Lage eine Dicke von mindestens 0,5 cm und höchstens 5,0 cm aufweist,
(iv) ein Messgerät zur Bestimmung des aus dem Inneren des ersten Hohlkörpers abgesaugten Fluidvolumens, beispielsweise eine Waage.

Die Messvorrichtung ist zur Verwendung mit einer Unterdrucktherapie-Vorrichtung vorgesehen. Die Unterdrucktherapie-Vorrichtung umfasst eine Unterdruckquelle, einen Fluidbehälter zur Aufnahme des aus dem Inneren des zweiten Hohlkörpers abgesaugten Fluids, einen Unterdruckverband mit Unterdruckanschlussstück, sowie eine Drainageleitung.

Gemäß einer besonders bevorzugten Variante der Erfindung umfasst die Messvorrichtung eine Einrichtung zur kontinuierlichen Zuführung des zweiten Fluids. Eine derartige Messvorrichtung für Wundverbände umfasst
(i) mindestens einen mit einem ersten Fluid gefüllten ersten Hohlkörper mit flexibler Wandstruktur,
(ii) einen zweiten Hohlkörper mit einer flexiblen äußeren Hüllstruktur, wobei die äußere Hüllstruktur mindestens eine erste Öffnung aufweist, welche eine Wunde simulieren kann, und wobei die äußere Hüllstruktur mindestens eine zweite Öffnung aufweist, welche die Zufuhr eines zweiten Fluids in das Innere des zweiten Hohlkörpers ermöglicht,
   wobei weiterhin der mindestens ein erster Hohlkörper in das Innere des zweiten Hohlkörpers einbringbar ist,
(iii) optional eine zusätzliche Lage eines flexiblen Materials, welches die innere Seite der Hüllstruktur des zweiten Hohlkörpers zumindest teilweise auskleidet, wobei die zusätzliche Lage eine Dicke von mindestens 0,5 cm und höchstens 5,0 cm aufweist,
(iv) eine Fluidquelle, wobei optional Heiz- und/oder Kühlmittel vorgesehen sein können, um die Temperatur des Fluids auf einen gewünschten Wert einzustellen.
(v) ein Leitungsmittel, so dass eine Fluidverbindung zwischen der Fluidquelle und dem Inneren des zweiten Hohlkörpers herstellbar ist,
   wobei das Leitungsmittel entweder durch die zweite Öffnung in das Inneren des zweiten Hohlkörpers durchgeführt wird
   oder
   wobei das Leitungsmittel an ein Anschlussstück, welches an der zweiten Öffnung vorgesehen ist, angeschlossen wird,
(vi) ein Messgerät zur Bestimmung des aus dem Inneren des ersten Hohlkörpers abgesaugten Fluidvolumens, beispielsweise eine Waage.

Die Messvorrichtung ist zur Verwendung mit einer Unterdrucktherapie-Vorrichtung vorgesehen. Die Unterdrucktherapie-Vorrichtung umfasst eine Unterdruckquelle, einen Fluidbehälter zur Aufnahme des aus dem Inneren des zweiten Hohlkörpers abgesaugten Fluids, einen Unterdruckverband mit Unterdruckanschlussstück, sowie eine Drainageleitung.

Des Weiteren kann im Inneren des zweiten Hohlkörpers eine Heiz- und/oder Kühlvorrichtung vorgesehen sein, wobei die Heiz- und/oder Kühlmittel über eine vierte Öffnung des zweiten Hohlkörpers eingebracht werden.

### Figurenbeschreibung

Fig. 1 zeigt eine schematische Darstellung der erfindungsgemäßen Vorrichtung zur Simulation einer Wunde am Körper eines Säugetiers, insbesondere zur Simulation einer Wunde am offenen Abdomen, im Querschnitt.
Fig. 2 zeigt Fig. 1 zeigt eine schematische Darstellung einer weiteren Ausführungsform der erfindungsgemäßen Vorrichtung im Querschnitt.
Fig. 3 zeigt eine schematische Darstellung einer Messvorrichtung für Wundverbände einschließlich eines zu testenden NPWT-Abdominalverband im Querschnitt.
Fig. 4 zeigt die bei einem Simulationsexperiment von drei unterschiedlichen getesteten NPWT-Verbänden aufgenommenen Volumina an Blutersatzlösung.

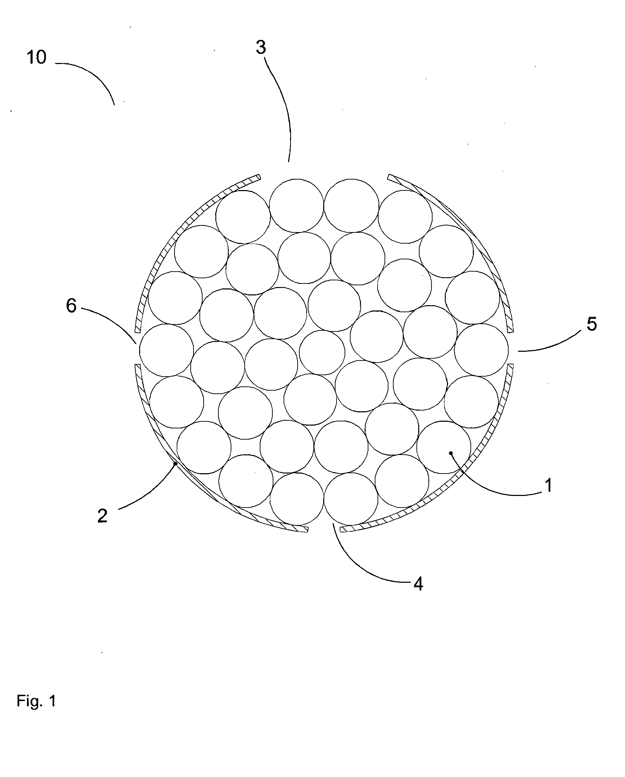

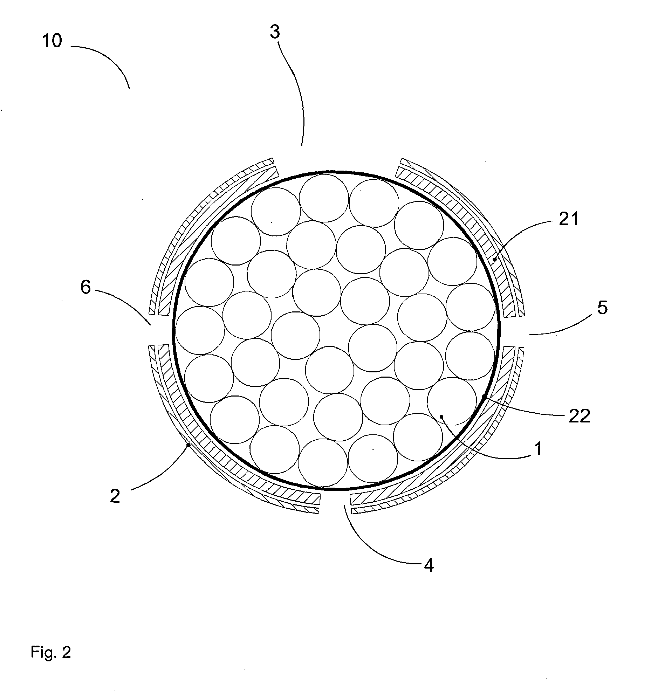

In **Figur 1** ist eine Ausführungsform einer Vorrichtung (10) zur Simulation einer Wunde am Körper eines Säugetiers, insbesondere zur Simulation einer Wunde am offenen Abdomen, dargestellt. Die Vorrichtung umfasst einen einzigen zweiten Hohlkörper (2) mit einer flexiblen äußeren Hüllstruktur und eine Vielzahl erster Hohlkörper (1) mit flexibler Wandstruktur. Die Hohlkörper (1) sind mit einem ersten Fluid gefüllt und Fluid-dicht verschlossen, so dass das Fluid nicht aus dem Hohlkörper (1) austreten kann. Eine erste Öffnung (3) in der äußeren Hüllstruktur des Hohlkörpers (2) bildet eine künstliche Wunde, wobei der die erste Öffnung (3) umgebende Rand des Hohlkörpers (2) den Wundrand simuliert. Größe und Umrissform der Öffnung (3) sollten dabei der zu simulierenden Wunde entsprechen. Der künstliche Wundgrund wird durch die Oberfläche der ersten Hohlkörper (1) im Bereich der ersten Öffnung (3) gebildet. In den zweiten Hohlkörper (2) können dann beispielsweise ca. 50 mit Wasser gefüllte erste Hohlkörper (1) eingebracht werden. Die mit Wasser gefüllten ersten Hohlkörper (1) können sich innerhalb des zweiten Hohlkörpers (2) in einer zusätzlichen Hülle befinden (in Figur 1 nicht gezeigt, siehe Figur 2). Als zusätzliche Hülle kann eine Kunststofffolie geeigneter Größe verwendet werden. Eine derartige Vorrichtung kann als Wundsimulator zur Testung von Wundauflagen verwendet werden. Zur Testung der Drainagekapazität eines Verbandes muss vor der Applikation des Verbandes eine gewünschte Menge einer Blutersatzlösung über die erste Öffnung (3) in das Innere des zweiten Hohlkörpers (2) gegossen werden. Die Verteilung der Blutersatzlösung innerhalb des zweiten Hohlkörpers (2) kann überwacht werden, wenn ein zweiter Hohlkörper (2) mit einer transparenten Hülle verwendet wird.

Die in **Figur 2** gezeigte Ausführungsform der Vorrichtung (10) weist über den in Figur 1 dargestellten Elementen hinaus zusätzliche optionale Merkmale auf.

Die Außenhülle des zweiten Hohlkörpers (2) umfasst weitere Öffnungen (4, 5 und 6).

Es ist eine zweite Öffnung (4) vorgesehen, so dass eine Blutersatzlösung kontinuierlich und auch bei angelegtem Testverband in das Innere des zweiten Hohlkörpers (2) geleitet werden kann. Eine kontinuierliche Zuleitung einer Blutersatzlösung ermöglicht eine besonders realistische Simulation einer stark exsudierenden Wunde.

Weiterhin ist eine dritte Öffnung (5) vorgesehen, die einen Anschluss von im Inneren des zweiten Hohlkörpers (2) angeordneten Sensoren (in Figur 2 nicht dargestellt) ermöglicht. Vorzugsweise dient die dritte Öffnung (5) dem Anschluss eines Druck- und/oder Temperatursensors. Bei der Durchführung von Leitungen durch die dritte Öffnung (5) muss eine luft- und flüssigkeitsundurchlässige Abdichtung hergestellt werden. Falls bei einem Simulationsexperiment keine Leitung durch eine dritte Öffnung (5) geführt werden, muss die Öffnung (5) dichtend abgeklebt werden.

Eine vierte Öffnung (6) ist zur Durchführung eines Heiz- und/oder Kühlelementes (in Figur 2 nicht dargestellt) in das Innere des zweiten Hohlkörpers (2) vorgesehen, so dass eine Temperierung der Vorrichtung (10) ermöglicht werden kann. Bei der Durchführung von Heiz- und/oder Kühlleitungen durch die vierte Öffnung (6) muss gleichfalls eine luft- und flüssigkeitsundurchlässige Abdichtung hergestellte werden. Werden keine Heiz- und/oder Kühlleitungen durch die vierte Öffnung (6) geführt, so muss die Öffnung (6) dichtend abgeklebt werden.

Des Weiteren wurde die Innenseite der Hülle des zweiten Hohlkörpers (2) mit einer zusätzlichen Lage eines flexiblen Materials (21) ausgekleidet. Hierzu wurde eine Schaumstoffmatte aus Polyurethan mit einer Dicke von 1 cm eingesetzt. Aus der Schaumstoffmatte (21) wurden durch Schneiden geeignete Abschnitte hergestellt, die auf die Innenseite des zweiten Hohlkörpers (2) geklebt wurden. Alle vorhandenen Öffnungen (3, 4, 5 und 6) müssen dabei durch die Schaumstofflage (21) gehen, d.h. die Innenverkleidung muss im Bereich der vorgenannten Öffnungen entfernt werden.

Die mit einem ersten Fluid gefüllten ersten Hohlkörper (1) wurden in Ihrer Gesamtheit in eine zusätzliche sackartige Umhüllung (22) eingebracht, wobei die zusätzliche Umhüllung aus einem flexiblen Folienmaterial mit einer Dicke von ca. 0,5 mm besteht. Eine derartige zusätzliche Umhüllung (22) kann insbesondere dann vorteilhaft sein, wenn eine Vielzahl erster Hohlkörper (1) im Inneren des zweiten Hohlkörpers (2) vorhanden ist. Im Bereich der künstlichen Wunde (3) ergibt sich durch die in ihrer Gesamtheit mit einer zusätzliche Umhüllung (22) umfassten Hohlkörper (1) eine Oberflächenbeschaffenheit, die hinsichtlich Relief und Festigkeit einer realen Wunde im Abdominalbereich vergleichbar ist.

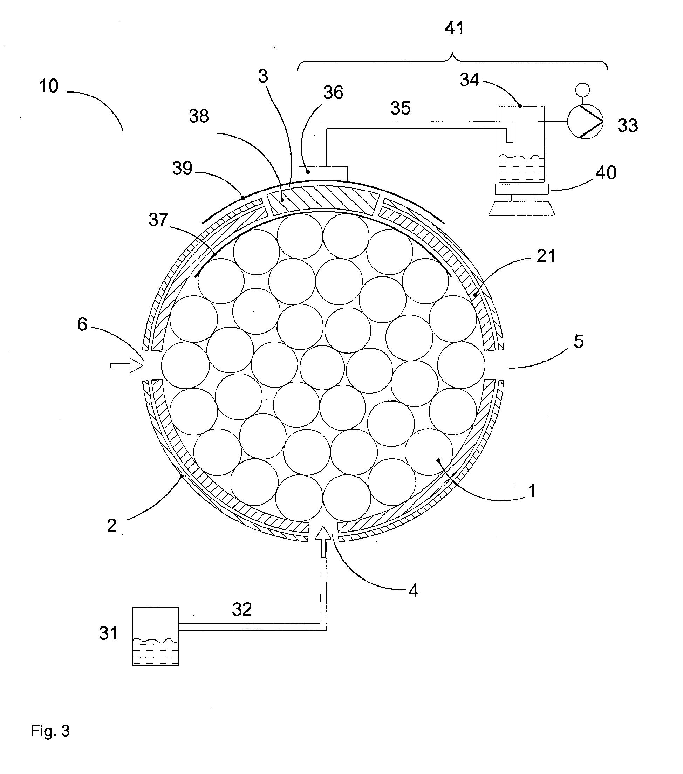

**Figur 3** zeigt eine Ausführungsform einer vollständigen Messvorrichtung (30) für Wundverbände mit angelegtem NPWT-Abdominalverband. Der zweite Hohlkörper (2) weist eine transparente Hülle auf. In den zweiten Hohlkörpers (2) wurde eine Vielzahl von mit Wasser gefüllten ersten Hohlkörpers (1) eingebracht. Die äußere Hüllstruktur des zweiten Hohlkörpers (2) weist eine die Wunde simulierende erste Öffnung (3), sowie eine zweite Öffnung (4) auf, welche eine kontinuierliche Zufuhr einer Blutersatzlösung in das Innere des zweiten Hohlkörpers ermöglicht. Die innere Seite der Hülle des zweiten Hohlkörpers (2) wurde mit einer zusätzlichen Lage eines flexiblen Materials (21) ausgekleidet. Bei dem flexiblen Material handelt es sich um eine Schaumstoffmatte aus Polyurethan mit einer Dicke von 1 cm. Die Schaumstoffmatte wurde an die Innenseite des zweiten Hohlkörpers (2) geklebt. Sie könnte jedoch auch lose eingelegt werden. Weiterhin ist eine Fluidquelle (31) vorgesehen, so dass Blutersatzlösung über die Leitung (32) und über die zweite Öffnung (4) in das Innere des zweiten Hohlkörpers (2) geleitet werden kann. Die Fluidquelle (31) umfasst einen Vorratsbehälter mit Blutersatzlösung und eine daran gekoppelte Pumpe (in Figur 3 nicht dargestellt). Das Leitungsmittel (32) wird durch die zweite Öffnung (4) in das Inneren des zweiten Hohlkörpers (2) geführt. Alternativ kann das Leitungsmittel (32) an ein geeignetes Anschlussstück (in Figur 3 nicht dargestellt), welches an der zweiten Öffnung (4) vorgesehen ist, angeschlossen werden.

Die Messvorrichtung umfasst weiterhin ein NPWT-System (Unterdrucktherapieeinrichtung) mit einer Unterdruckquelle (33), einen Sammelkanister (34) zur Aufnahme des aus dem Inneren des zweiten Hohlkörpers (2) abgesaugten Fluids und einer Drainageleitung (35), die zum Anschluss an das Unterdruckanschlussstück (Port; 36) eines Unterdrucktherapieverbandes vorgesehen ist. Der in Figur 3 dargestellte Abdominalverband umfasst einen Folienanteil (37), eine Schaumstofflage (38), eine Abdeckfolie (39) und ein Unterdruckanschlussstück (36). Der Folienanteil (37) des Verbands liegt dem künstlichen Wundgrund (gebildet durch die ersten Hohlkörper (1)) im Sinne einer Wundkontaktschicht direkt auf. Der Folienanteil (37) wird in den durch Bauchdecke (simuliert durch den zweiten Hohlkörper (2)) und Gedärme (simuliert durch den ersten Hohlkörpers (1)) gebildeten Zwischenraum eingeschoben. Zur Erhöhung der Drainagekapazität des Verbandes können auf den Folienanteil (37) Leitungsmittelabschnitte in sternförmiger Anordnung aufgebracht werden (in Figur 3 nicht dargestellt). Der Abdominalverband umfasst weiterhin eine Schaumstofflage (38) aus einem offenzelligen Polyurethanschaumstoff. Umfang und Dicke des Schaumstoffs (38) sollten möglichst exakt an die künstliche Wunde (erste Öffnung 3 im zweiten Hohlkörpers (2)) angepasst werden, so dass Schaumstoffränder und Wundränder idealerweise auf Stoss zu liegen kommen. Über die künstliche Wunde (3) wird eine luftdichte selbstklebende Abdeckfolie (39) aus einem Polyurethanfilm platziert und außerhalb des Bereichs der künstlichen Wunde an der Oberfläche des zweiten Hohlkörpers (2) fixiert. Die Messvorrichtung (40), bei der es sich um eine elektronische Waage handelt, dient zur Bestimmung des aus dem Inneren des zweiten Hohlkörpers (2) abgesaugten Fluidvolumens. Idealerweise weist die Messvorrichtung (40) einen Computeranschluss auf, so dass die Messwerte während des Simulationsexperimentes kontinuierlich aufgezeichnet werden können. Weiterhin ist eine dritte Öffnung (5) am zweiten Hohlkörper (2) vorgesehen. Über diese Öffnung können weitere Messgeräte, beispielsweise ein Druckmesser oder ein Temperatursensor in das Innere des zweiten Hohlkörpers (2) eingeführt werden. Optional können zusätzliche Drucksensoren im NPWT-Verband vorgesehen sein (in Figur 3 nicht dargestellt).

Durch eine optional vorhandene vierte Öffnung (6) können bei Bedarf Heiz- und/oder Kühlmittel (in Figur 3 nicht dargestellt) in das Innere des zweiten Hohlkörpers (2) geführt werden, um die Innentemperatur des Wundsimulators auf einen gewünschten Wert einzustellen.

Die vorliegende Erfindung umfasst gleichfalls ein Verfahren zur Herstellung einer Vorrichtung (10; 20) zur Simulation einer Wunde am Körper eines Säugetiers, insbesondere zur Simulation einer Wunde am offenen Abdomen, umfassend
(i) Bereitstellen mindestens eines mit einem ersten Fluid gefüllten ersten Hohlkörpers (1) mit flexibler Wandstruktur,
(ii) Bereitstellen eines zweiten Hohlkörpers (2) mit einer flexiblen äußeren Hüllstruktur, wobei die äußere Hüllstruktur mindestens eine erste Öffnung (3) aufweist, welche eine Wunde simulieren kann,
   und wobei der mindestens eine erste Hohlkörper (1) in das Innere des zweiten Hohlkörpers (2) einbringbar ist.
(iii) Füllen des mindestens einen mit einem ersten Fluid gefüllten ersten Hohlkörpers (1) mit einem Fluid, insbesondere mit Wasser oder Luft,
(iv) Verschließen des mindestens einen Hohlkörpers (1), so dass kein Fluid aus dem Inneren des Hohlkörpers (1) austreten kann,
(v) Einbringen des mindestens einen mit einem Fluid gefüllten ersten Hohlkörpers (1) in das Innere des zweiten Hohlkörpers (1), so dass das Innere des zweiten Hohlkörpers (2) weitestgehend gefüllt ist.

Weiterhin offenbart die Erfindung ein Verfahren zur Messung der Drainagekapazität eines zur Unterdrucktherapie von Wunden vorgesehenen Verbandes. Das Verfahren umfasst die Schritte:
(i) Bereitstellen einer Messvorrichtung (30) umfassend mindestens einen mit einem ersten Fluid gefüllten ersten Hohlkörpers (1) mit flexibler Wandstruktur, einen zweiten Hohlkörpers (2) mit einer flexiblen äußeren Hüllstruktur, wobei die äußere Hüllstruktur mindestens eine erste Öffnung (3) und eine zweite Öffnung (4) aufweist, eine Fluidquelle (31) und ein Leitungsmittel (32) für die Zuleitung der Blutersatzlösung.
(ii) Bereitstellen eines NPWT-Systems (Unterdrucktherapieeinrichtung) mit einer Unterdruckquelle (33), einen Sammelkanister (34) zur Aufnahme des aus dem Inneren des zweiten Hohlkörpers (2) abgesaugten Fluids und einer Drainageleitung (35), die zum Anschluss an das Unterdruckanschlussstück (36) eines Unterdrucktherapieverbandes vorgesehen ist.
(iii) Bereitstellen des zu testenden NPWT-Verbandes.
(iv) Herstellung einer Fluidverbindung zwischen einer Fluidquelle (31) und dem Inneren des zweiten Hohlkörpers (2) unter Verwendung des Leitungsmittel (32),
   wobei das Leitungsmittel (32) entweder durch die zweite Öffnung (4) in das Inneren des ersten Hohlkörpers (1) durchgeführt wird
   oder
   wobei das Leitungsmittel an ein Anschlussstück, welches an der zweiten Öffnung (4) vorgesehen ist, angeschlossen wird.
(v) Anlegen des zu testenden Verbandes an die erste Öffnung (künstliche Wunde) (3).
(vi) Applikation der Unterdrucktherapieeinrichtung an den zu testenden Verband.
(vii) Zufuhr eines gewünschten Fluid-Volumens in das Innere des zweiten Hohlkörpers (2) und Inbetriebnahme der Unterdrucktherapieeinrichtung.
(viii) Bestimmung des pro Zeiteinheit aus dem Inneren des zweiten Hohlkörpers (2) abgesaugten Fluid-Volumens.
(ix) Optional Erfassung weiterer Messdaten wie beispielsweise Innendruck Pᵢ und Innentemperatur Tᵢ.
(x) Optional die Erfassung und Verarbeitung der Messdaten mittels eines Computers.

### Anwendungsbeispiel

Testung von Abdominalwundauflagen unter Verwendung eines NPWT-Wundsimulators.

In einen transparenten Ball aus PVC (zweiter Hohlkörper) mit einem Volumen von 15.187 cm³ und mit einer Wandstärke von 1,6 mm wurde zur Simulation einer Wunde ein ellipsenförmiges Loch mit einer offenen Fläche von etwa 295 cm² geschnitten. Der Ball wurde so gedreht, dass die derart hergestellte künstliche Wunde (erste Öffnung) nach oben zeigte.

In ca. 50 Hohlkörper aus Gummi und mit einer Wandstärke von 0,3 mm wurde jeweils ca. 268 ml Wasser gefüllt. Die Hohlkörper wurden Fluid-dicht verschlossen.

Ein Vakuumbeutel aus Polyethylen mit einer Wandstärke von ca. 1 mm wurde über die ellipsenförmige erste Öffnung des Balls in das Innere des PVC-Balls eingebracht. Der Vakuumbeutel war so dimensioniert, dass der gesamte Innenraum des PVC-Balls damit ausgekleidet werden konnte. Die mit Wasser gefüllten Hohlkörper aus Gummi wurden in ihrer Gesamtheit über die erste Öffnung des PVC-Balls in den im Inneren des PVC-Balls befindlichen Vakuumbeutel gelegt, d.h. die Hohlkörper aus Gummi befanden sich sowohl im Inneren des PVC-Balls als auch innerhalb des Vakuumbeutels als zusätzliche Hülle. Die im Vakuumbeutel befindlichen und mit Wasser gefüllten Hohlkörper aus Gummi füllten den inneren Hohlraum PVC-Balls weitestgehend aus. Der mit Hohlkörpern aus Gummi gefüllte Vakuumbeutel wurde durch Umfalten seines Randes verschlossen.

Die zu testenden Abdominalwundauflagen wurden wie folgt in die künstliche Wunde appliziert:
Wundauflage 1: Wundauflage ABThera™ des Herstellers KCI (USA). Das Grundelement der ABThera™ Wundauflage ist eine geschlitzte Polyurethanfolie. Auf die Folie sind in sternförmiger Anordnung segmentierte Schaumstoffstreifen aufgebracht. Auf der im Gebrauch von der Wunde abgewandten Seite der Anordnung aus Folie und Schaumstoffstreifen befindet sich eine weitere Lage einer geschlitzten Folie. Im zentralen Bereich der Wundauflage wurde eine weitere Schaumstofflage derart aufgelegt, dass die Ränder der weiteren Schaumstofflage in direktem Kontakt, d.h. auf Stoss, mit den Rändern der künstlichen Wunde (erste Öffnung, welche in den PVC-Ball geschnitten wurde) stehen. Die künstliche Wunde wurde mit einer luftdichten Abdeckfolie abgedeckt, wobei die Abdeckfolie auf die äußere Oberfläche des PVC-Balls in der Umgebung der künstlichen Wunde geklebt wurde. Über ein Unterdruckanschlussstück (Port) wurde eine Unterdruckkommunikation zwischen dem Wundraum der künstlichen Wunde und einer Unterdruckquelle ATMOS S041 (Atmos, Deutschland) hergestellt.
Wundauflage 2: Prototyp für eine Abdominalwundauflage. Auf eine geschlitzte Polyurethanfolie wurden in sternförmiger Anordnung 6 JP-Flachdrainage-Schläuche (Primed, Deutschland) aufgebracht, wobei die Drainage-Schläuche über die gesamte Länge Perforationen (Durchmesser ca. 1 bis 2 mm) aufweisen. Auf der im Gebrauch von der Wunde abgewandten Seite der Anordnung aus Drainageschläuchen und erster Folienlage wurde eine zweite Lage einer Polyurethanfolie, welche neben einer zentralen Öffnung zur Ableitung des Wundexsudates keine weiteren Öffnungen aufweist, aufgebracht. Im zentralen Bereich der Wundauflage wurde wie bei Wundauflage 1 beschrieben eine weitere Schaumstofflage derart aufgebracht, dass die Ränder der weiteren Schaumstofflage in direktem Kontakt, d.h. auf Stoss, mit den Rändern der künstlichen Wunde (Öffnung, welche in den PVC-Ball geschnitten wurde) stehen. Die luftdichte Abdeckung der künstlichen Wunde und der Anschluss an eine Unterdruckquelle erfolgten wie vorstehend unter Wundauflage 1 beschrieben.
Wundauflage 3: Alternativer Prototyp für eine Abdominalwundauflage. Auf eine perforierte Polyethylenfolie (offene Fläche der Öffnung ca. 14 % bezogen auf die gesamte Fläche der Folie; Type 4460 von rkw, Deutschland) wurden in sternförmiger Anordnung 6 mehrlumige trapezförmige Flachdrainage-Schlauchabschnitte unlösbar aufgebracht, wobei jeder der Leitungsmittelabschnitte mit Ausnahme der endständigen Öffnungen über keine weiteren Öffnungen verfügt. Auf der im Gebrauch von der Wunde abgewandten Seite der Anordnung aus Drainageschläuchen und erster Folienlage wurde eine zweite Lage einer Polyurethanfolie, welche neben einer zentralen Öffnung zur Ableitung des Wundexsudates keine weiteren Öffnungen aufweist, aufgebracht. Im zentralen Bereich der Wundauflage wurde wie bei Wundauflage 1 und 2 beschrieben eine weitere Schaumstofflage lose aufgebracht. Die luftdichte Abdeckung der künstlichen Wunde und der Anschluss an eine Unterdruckquelle erfolgten wie vorstehend unter Wundauflage 1 beschrieben.

Die Versuchsdauer betrug jeweils 60 min. Es wurde ein konstanter Unterdruck von 125 mm Hg angelegt.

Über eine an die Ventilöffnung des Balls angeschlossene Leitung wurde über den Versuchszeitraum von 60 min insgesamt ca. 500 ml einer gefärbten Blutersatzlösung kontinuierlich und mit gleich bleibender Flussrate eingebracht. Zur Herstellung eines Liters der Blutersatzlösung wurden 566 g demineralisiertes Wasser, 425 g Glycerin, 9 g NaCl und 0,2 g des Farbstoffs Allura Red vermischt. Die Lösung weist bei 23°C eine Viskosität von ca. 4,5 mPa*s auf. Die über das NPWT-System abgepumpte Blutersatzlösung wurde im Kanister der Unterdruckeinheit gesammelt. Das Volumen der abgesaugten Blutersatzlösung wurde durch Wiegen bestimmt. Der Versuch wurde bei Raumtemperatur durchgeführt (23°C). Eine Temperierung der Blutersatzlösung erfolgte nicht, d.h. die Blutersatzlösung hatte gleichfalls eine Temperatur von etwa 23°C.

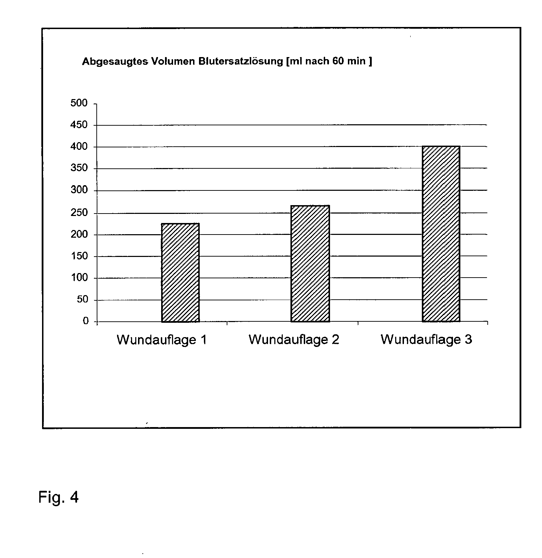

Es wurde beobachtet, dass die Blutersatzlösung bei den Wundauflagen 1 und 2 nicht in den durch die Folienlagen gebildeten Zwischenraum eindringt. Stattdessen scheint die Blutersatzlösung zwischen den künstlichen Gedärmen (Oberfläche der mit einer zusätzlichen Hülle umgebenen Hohlkörper aus Gummi) und der wundseitigen Oberfläche der geschlitzten Folie zum Zentrum der Wundauflage zu fließen und dort abgesaugt zu werden. Dagegen wurde bei der Wundauflage 3 beobachtet, dass die Blutersatzlösung in den zwischen der perforierten Folie (wundseitig) und der nicht-perforierten weiteren Folienlage gebildeten Zwischenraum eindringt. Der Weitertransport der Blutersatzlösung aus dem Folien-Zwischenraum zur Unterdruckquelle wurde möglicherweise durch Kapillareffekte begünstigt. Insgesamt wurde bei dem mit Wundauflage 3 durchgeführtem Experiment im Vergleich zu den mit den Wundauflagen 1 und 2 durchgeführten Experimenten ein größeres Volumen an Blutersatzlösung in die Unterdruckquelle gesaugt (siehe auch Fig. 4):
Wundauflage 1: 245 ml Blutersatzlösung in 60 min abgesaugt
Wundauflage 2: 265 ml Blutersatzlösung in 60 min abgesaugt
Wundauflage 3: 400 ml Blutersatzlösung in 60 min abgesaugt

Die Versuchsanordnung erlaubt einen Vergleich unterschiedlicher Abdominalwundauflagen hinsichtlich ihrer jeweiligen Drainagekapazität. Die in einer zusätzlichen Hülle eingebrachten und mit Wasser gefüllten Hohlkörper aus Gummi bilden einen künstlichen Wundgrund, welcher hinsichtlich seiner Oberflächenbeschaffenheit dem Wundgrund, der bei einer realen Abdominalwunde durch die freigelegten Gedärmen entsteht, sehr ähnlich ist.

## Patentansprüche

1. Vorrichtung (10) zur Simulation einer Wunde am Körper eines Säugetiers, insbesondere zur Simulation einer Wunde am offenen Abdomen, umfassend
(i) mindestens einen mit einem ersten Fluid füllbaren ersten Hohlkörper (1) mit flexibler Wandstruktur,
und
(ii) einen zweiten Hohlkörper (2) mit einer flexiblen äußeren Hüllstruktur, wobei die äußere Hüllstruktur mindestens eine erste Öffnung (3) zum Simulieren einer Wunde aufweist,
und wobei der mindestens eine erste Hohlkörper (1) in das Innere des zweiten Hohlkörpers (2) einbringbar ist.

2. Vorrichtung (10) nach Anspruch 1, wobei der zweite Hohlkörper (2) mindestens eine zweite Öffnung (4) aufweist, welche die Zufuhr eines zweiten Fluids in das Innere des zweiten Hohlkörpers (2) ermöglicht.

3. Vorrichtung (10) nach Anspruch 1 oder 2, wobei die Vorrichtung mindestens 3 und höchstens 1.000 der ersten Hohlkörper (1), vorzugsweise mindestens 25 und höchstens 150 der ersten Hohlkörper (1), welche in das Innere des zweiten Hohlkörpers (2) einbringbar sind, umfasst.

4. Vorrichtung (20) nach einem der vorangehenden Ansprüche, wobei die Vorrichtung weiterhin eine zusätzliche Lage eines flexiblen Materials (21) zur Auskleidung zumindest eines Anteils der inneren Seite der Hüllstruktur des zweiten Hohlkörpers (2) umfasst, und wobei die zusätzliche Lage (21) eine Dicke von mindestens 0,5 cm und höchstens 5,0 cm aufweist.

5. Vorrichtung (10; 20) nach einem der vorangehenden Ansprüche, wobei der zweite Hohlkörper (2) mindestens eine dritte Öffnung (5) aufweist, welche zum Anschluss eines Drucksensors und/oder eines Temperatursensors vorgesehen ist, so dass der im Inneren des zweiten Hohlkörpers (2) vorhandene Innendruck Pᵢ und/oder die im Inneren des zweiten Hohlkörpers (2) vorhandene Temperatur Tᵢ bestimmt werden kann.

6. Vorrichtung (10; 20) nach einem der vorangehenden Ansprüche, wobei der zweite Hohlkörper (2) ein Volumen von mindestens 1.500 cm3 und höchstens 600.000 cm3 aufweist

7. Vorrichtung (10; 20) nach einem der vorangehenden Ansprüche, wobei der zweite Hohlkörper (2) eine transparente Hüllstruktur aufweist.

8. Vorrichtung (10; 20) nach einem der vorangehenden Ansprüche, wobei der zweite Hohlkörper (2) mindestens eine vierte Öffnung (6) aufweist, welche zum Anschluss eines Heiz- und/oder eines Kühlelementes vorgesehen ist, so dass die im Inneren des zweiten Hohlkörpers (2) vorhandene Temperatur eingestellt werden kann.

9. Vorrichtung (10; 20) nach einem der vorangehenden Ansprüche 2 bis 8, wobei die Vorrichtung eine außerhalb der Vorrichtung angeordnete Heiz- und/oder Kühleinrichtung umfasst, so dass die Temperatur des in das Innere des ersten Hohlkörpers (1) eingeleiteten ersten Fluids auf einen gewünschten Wert eingestellt werden kann.

10. Vorrichtung (20) nach einem der vorangehenden Ansprüche 3 bis 9, wobei die mit einem ersten Fluid gefüllten ersten Hohlkörper (1) in ihrer Gesamtheit in eine zusätzliche sackartige Umhüllung (22) eingebracht vorliegen, wobei die zusätzliche Umhüllung aus einem flexiblen Folienmaterial mit einer Dicke von weniger als 2 mm besteht.

11. Vorrichtung (20) nach einem der vorangehenden Ansprüche 4 bis 10, wobei es sich bei der zusätzlichen Lage des die innere Seite der Hüllstruktur des zweiten Hohlkörpers (2) zumindest teilweise auskleidenden Materials (21) um eine Lage aus einem geschäumten oder ungeschäumten elastomeren Kunststoff handelt.

12. Messvorrichtung (30) für Wundverbände, insbesondere für Wundverbände, welche zur Unterdrucktherapie von Wunden am offenen Abdomen vorgesehen sind, umfassend
(i) mindestens einen mit einem ersten Fluid füllbaren ersten Hohlkörper (1) mit flexibler Wandstruktur,
(ii) einen zweiten Hohlkörper (2) mit einer flexiblen äußeren Hüllstruktur, wobei die äußere Hüllstruktur mindestens eine erste Öffnung (3) zum Simulieren einer Wunde aufweist, und wobei die äußere Hüllstruktur mindestens eine zweite Öffnung (4) aufweist, welche die Zufuhr eines zweiten Fluids in das Innere des zweiten Hohlkörpers ermöglicht,
wobei weiterhin der mindestens eine erste Hohlkörper (1) in das Innere des zweiten Hohlkörpers (2) einbringbar ist,
(iii) optional eine zusätzliche Lage eines flexiblen Materials (21), welches die innere Seite der Hüllstruktur des zweiten Hohlkörpers (2) zumindest teilweise auskleidet, wobei die zusätzliche Lage eine Dicke von mindestens 0,5 cm und höchstens 5,0 cm aufweist,
(iv) eine Fluidquelle (31), wobei optional Heiz- und/oder Kühlmittel vorgesehen sein können, um die Temperatur des Fluids auf einen gewünschten Wert einzustellen,
(v) und ein Leitungsmittel (32), so dass eine Fluidverbindung zwischen der Fluidquelle und dem Inneren des zweiten Hohlkörpers (2) herstellbar ist,
wobei das Leitungsmittel entweder durch die zweite Öffnung (4) in das Inneren des zweiten Hohlkörpers (2) durchgeführt wird
oder
wobei das Leitungsmittel an ein Anschlussstück, welches an der zweiten Öffnung (4) vorgesehen ist, angeschlossen wird,
und
(vi) ein Messgerät zur Bestimmung des aus dem Inneren des ersten Hohlkörpers abgesaugten Fluidvolumens.

13. Verfahren zur Herstellung einer Vorrichtung (10; 20) nach einem der Ansprüche 1 bis 11, umfassend
(i) Bereitstellen mindestens eines mit einem ersten Fluid füllbaren ersten Hohlkörpers (1) mit flexibler Wandstruktur,
(ii) Bereitstellen eines zweiten Hohlkörpers (2) mit einer flexiblen äußeren Hüllstruktur, wobei die äußere Hüllstruktur mindestens eine erste Öffnung (3) aufweist, welche eine Wunde simulieren kann,
und wobei der mindestens eine erste Hohlkörper (1) in das Innere des zweiten Hohlkörpers (2) einbringbar ist,
(iii) Füllen des mindestens einen mit einem ersten Fluid gefüllten ersten Hohlkörpers (1) mit einem Fluid, insbesondere mit Wasser oder Luft,
(iv) Verschließen der Vielzahl zweiter Hohlkörper (2), so dass kein Fluid aus dem Inneren der Hohlkörper austreten kann,
(v) Einbringen des mindestens einen mit einem Fluid gefüllten ersten Hohlkörpers (1) in das Innere des zweiten Hohlkörpers (2), so dass das Innere des zweiten Hohlkörpers (2) weitestgehend gefüllt ist.

14. Verfahren zur Messung der Drainagekapazität eines zur Unterdrucktherapie von Wunden vorgesehenen Verbandes, umfassend
(i) Bereitstellen einer Messvorrichtung (30) nach Anspruch 12,
(ii) Herstellung einer Fluidverbindung (32) zwischen einer Fluidquelle (31) und dem Inneren des zweiten Hohlkörpers (2),
wobei das Leitungsmittel (32) entweder durch die zweite Öffnung (4) in das Inneren des ersten Hohlkörpers (1) durchgeführt wird
oder
wobei das Leitungsmittel an ein Anschlussstück, welches an der zweiten Öffnung (4) vorgesehen ist, angeschlossen wird,
(iii) Applikation einer Unterdrucktherapieeinrichtung (33; 34; 35; 36; 37; 38; 39), wobei der Unterdrucktherapieverbandes (36; 37; 38; 39) an der ersten Öffnung (3) angelegt wird,
(iv) Inbetriebnahme der Unterdrucktherapieeinrichtung und Zufuhr eines gewünschten Fluid-Volumens in das Innere des zweiten Hohlkörpers (2) und
(v) Bestimmung des pro Zeiteinheit aus dem Inneren des zweiten Hohlkörpers (2) abgesaugten Fluid-Volumens,
(vi) optional Erfassung von Innendruck Pi, Innentemperatur Ti und ggf. weiterer Messdaten,
(vii) optional die Erfassung und Verarbeitung der Messdaten mittels eines Computers.

## Claims

1. Device (10) for simulating a wound on the body of a mammal, in particular for simulating a wound on the open abdomen, comprising
(i) at least one first hollow body (1) which has a flexible wall structure and can be filled with a first fluid,
and
(ii) a second hollow body (2) with a flexible outer shell structure, wherein the outer shell structure has at least one first opening (3) for simulating a wound,
and wherein the at least one first hollow body (1) can be introduced into the interior of the second hollow body (2).

2. Device (10) according to Claim 1, wherein the second hollow body (2) has at least one second opening (4), which permits the supply of a second fluid into the interior of the second hollow body (2).

3. Device (10) according to Claim 1 or 2, wherein the device comprises at least 3 and at most 1,000 of the first hollow bodies (1), preferably at least 25 and at most 150 of the first hollow bodies (1), which can be introduced into the interior of the second hollow body (2).

4. Device (20) according to one of the preceding claims, wherein the device moreover has an additional layer of a flexible material (21) for lining at least a portion of the inner face of the shell structure of the second hollow body (2), and wherein the additional layer (21) has a thickness of at least 0.5 cm and at most 5.0 cm.

5. Device (10; 20) according to one of the preceding claims, wherein the second hollow body (2) has at least one third opening (5), which is provided for the attachment of a pressure sensor and/or of a temperature sensor, such that the internal pressure Pᵢ in the interior of the second hollow body (2) and/or the temperature Tᵢ in the interior of the second hollow body (2) can be determined.

6. Device (10; 20) according to one of the preceding claims, wherein the second hollow body (2) has a volume of at least 1,500 cm³ and at most 600,000 cm³.

7. Device (10; 20) according to one of the preceding claims, wherein the second hollow body (2) has a transparent shell structure.

8. Device (10; 20) according to one of the preceding claims, wherein the second hollow body (2) has at least one fourth opening (6), which is provided for the attachment of a heating and/or a cooling element, such that the temperature in the interior of the second hollow body (2) can be adjusted.

9. Device (10; 20) according to one of the preceding Claims 2 to 8, wherein the device comprises a heating and/or cooling means arranged outside the device, such that the temperature of the first fluid introduced into the interior of the first hollow body (1) can be adjusted to a desired value.

10. Device (20) according to one of the preceding Claims 3 to 9, wherein the first hollow bodies (1) filled with a first fluid are located in their entirety in an additional bag-like covering (22), wherein the additional covering is made of a flexible film material with a thickness of less than 2 mm.

11. Device (20) according to one of the preceding Claims 4 to 10, wherein the additional layer of the material (21) at least partially lining the inner face of the shell structure of the second hollow body (2) is a layer made of a foamed or unfoamed elastomer.

12. Measuring device (30) for wound dressings, in particular for wound dressings provided for negative-pressure therapy of wounds on the open abdomen, comprising
i) at least one first hollow body (1) which has a flexible wall structure and can be filled with a first fluid,
and
(ii) a second hollow body (2) with a flexible outer shell structure, wherein the outer shell structure has at least one first opening (3) for simulating a wound, and
wherein the outer shell structure has at least one second opening (4), which permits the supply of a second fluid into the interior of the second hollow body,
wherein furthermore the at least one first hollow body (1) can be introduced into the interior of the second hollow body (2),
(iii) optionally an additional layer of a flexible material (21) which at least partially lines the inner face of the shell structure of the second hollow body (2), wherein the additional layer has a thickness of at least 0.5 cm and at most 5.0 cm,
(iv) a fluid source (31), wherein heating and/or cooling means can optionally be provided in order to adjust the temperature of the fluid to a desired value,
(v) and a conduit (32), such that a fluid connection can be established between the fluid source and the interior of the second hollow body (2),
wherein the conduit is either routed through the second opening (4) into the interior of the second hollow body (2)
or
wherein the conduit is attached to a connector piece, which is provided on the second opening (4),
and
(vi) a measuring appliance for determining the volume of fluid aspirated from the interior of the first hollow body.

13. Method for producing a device (10; 20) according to one of Claims 1 to 11, comprising
(i) making available at least one first hollow body (1) which has a flexible wall structure and can be filled with a first fluid,
(ii) making available a second hollow body (2) with a flexible outer shell structure, wherein the outer shell structure has at least one first opening (3), which can simulate a wound,
and wherein the at least one first hollow body (1) can be introduced into the interior of the second hollow body (2),
(iii) filling the at least one first hollow body (1), which is filled with a first fluid, with a fluid, in particular with water or air,
(iv) closing the multiplicity of second hollow bodies (2) such that no fluid can escape from the interior of the hollow bodies,
(v) introducing the at least one first hollow body (1), filled with a fluid, into the interior of the second hollow body (2), such that the interior of the second hollow body (2) is very substantially filled.

14. Method for measuring the drainage capacity of a dressing provided for negative-pressure therapy of wounds, comprising
(i) making available a measuring device (30) according to Claim 12,
(ii) establishing a fluid connection (32) between a fluid source (31) and the interior of the second hollow body (2),
wherein the conduit (32) is either routed through the second opening (4) into the interior of the first hollow body (1)
or
wherein the conduit is attached to a connector piece, which is provided on the second opening (4),
(iii) applying a negative-pressure therapy appliance (33; 34; 35; 36; 37; 38; 39), wherein the negative-pressure therapy dressing (36; 37; 38; 39) is placed on the first opening (3),
(iv) starting up the negative-pressure therapy appliance and supplying a desired volume of fluid into the interior of the second hollow body (2) and
(v) determining the volume of fluid aspirated per unit of time from the interior of the second hollow body (2),
(vi) optionally detecting internal pressure Pᵢ, internal temperature Tᵢ and possibly other measurement data,
(vii) optionally detecting and processing the measurement data by means of a computer.

## Revendications

1. Dispositif (10) destiné à simuler une plaie sur un corps d'un mammifère, en particulier pour la simulation d'une plaie sur un abdomen ouvert, comprenant
(i) au moins un premier corps creux (1) avec une structure de paroi souple et pouvant être rempli avec un premier fluide,
et
(ii) un deuxième corps creux (2) avec une structure d'enveloppe extérieure souple, dans lequel la structure d'enveloppe extérieure présente au moins une première ouverture (3) destinée à simuler une plaie,
et dans lequel ledit au moins un premier corps creux (1) peut être introduit à l'intérieur du deuxième corps creux (2).

2. Dispositif (10) selon la revendication 1, dans lequel le deuxième corps creux (2) présente au moins une deuxième ouverture (4), qui permet l'introduction d'un deuxième fluide à l'intérieur du deuxième corps creux (2).

3. Dispositif (10) selon la revendication 1 ou 2, dans lequel le dispositif comprend au moins 3 et au plus 1.000 des premiers corps creux (1), de préférence au moins 25 et au plus 150 des premiers corps creux (1), qui peuvent être introduits à l'intérieur du deuxième corps creux (2).

4. Dispositif (20) selon l'une quelconque des revendications précédentes, dans lequel le dispositif comprend en outre une couche supplémentaire d'un matériau souple (21) destinée à recouvrir au moins une partie du côté intérieur de la structure d'enveloppe du deuxième corps creux (2), et dans lequel la couche supplémentaire (21) présente une épaisseur au moins de 0,5 cm et au plus de 5,0 cm.

5. Dispositif (10, 20) selon l'une quelconque des revendications précédentes, dans lequel le deuxième corps creux (2) présente au moins une troisième ouverture (5), qui est prévue pour le raccordement d'un capteur de pression et/ou d'un capteur de température, de telle manière que la pression interne Pᵢ régnant à l'intérieur du deuxième corps creux (2) et/ou la température Tᵢ régnant à l'intérieur du deuxième corps creux (2) puisse(nt) être déterminée(s)

6. Dispositif (10, 20) selon l'une quelconque des revendications précédentes, dans lequel le deuxième corps creux (2) présente un volume au moins de 1 500 cm³ et au plus de 600 000 cm³.

7. Dispositif (10, 20) selon l'une quelconque des revendications précédentes, dans lequel le deuxième corps creux (2) présente une structure d'enveloppe transparente.

8. Dispositif (10, 20) selon l'une quelconque des revendications précédentes, dans lequel le deuxième corps creux (2) présente au moins une quatrième ouverture (6), qui est prévue pour le raccordement d'un élément de chauffage et/ou de refroidissement, de telle manière que la température régnant à l'intérieur du deuxième corps creux (2) puisse être réglée.

9. Dispositif (10, 20) selon l'une quelconque des revendications précédentes 2 à 8, dans lequel le dispositif comprend un système de chauffage et/ou de refroidissement disposé à l'extérieur du dispositif, de telle manière que la température du premier fluide introduit à l'intérieur du premier corps creux (1) puisse être réglée à une valeur désirée.

10. Dispositif (20) selon l'une quelconque des revendications précédentes 3 à 9, dans lequel les premiers corps creux (1) remplis d'un premier fluide sont placés dans leur totalité dans une enveloppe supplémentaire en forme de sac (22), dans lequel l'enveloppe supplémentaire se compose d'un matériau en feuille souple d'une épaisseur de moins de 2 mm.

11. Dispositif (20) selon l'une quelconque des revendications précédentes 4 à 10, dans lequel la couche supplémentaire du matériau (21) recouvrant au moins partiellement le côté intérieur de la structure d'enveloppe du deuxième corps creux (2) est une couche en une matière plastique élastomère expansée ou non expansée.

12. Dispositif de mesure (30) pour des pansements de plaies, en particulier pour des pansements de plaies destinés à la thérapie en dépression de plaies sur un abdomen ouvert, comprenant
(i) au moins un premier corps creux (1) avec une structure de paroi souple et pouvant être rempli avec un premier fluide,
(ii) un deuxième corps creux (2) avec une structure d'enveloppe extérieure souple, dans lequel la structure d'enveloppe extérieure présente au moins une première ouverture (3) destinée à simuler une plaie, et dans lequel la structure d'enveloppe extérieure présente au moins une deuxième ouverture (4), qui permet l'introduction d'un deuxième fluide à l'intérieur du deuxième corps creux,
dans lequel en outre ledit au moins un premier corps creux (1) peut être introduit à l'intérieur du deuxième corps creux (2),
(iii) en option, une couche supplémentaire d'un matériau souple (21), qui recouvre au moins partiellement le côté intérieur de la structure d'enveloppe du deuxième corps creux (2), dans lequel la couche supplémentaire présente une épaisseur au moins de 0,5 cm et au plus de 5,0 cm,
(iv) une source de fluide (31), dans lequel en option des moyens de chauffage et/ou de refroidissement peuvent être prévus, afin de régler la température du fluide à une valeur désirée,
(v) et un moyen de conduite (32), de telle manière qu'une liaison de fluide puisse être établie entre la source de fluide et l'intérieur du deuxième corps creux (2),
dans lequel soit le moyen de conduite est mené à travers la deuxième ouverture (4) jusqu'à l'intérieur du deuxième corps creux (2),
soit
le moyen de conduite est raccordé à une pièce de raccord, qui est prévue à la deuxième ouverture (4),
et
(vi) un appareil de mesure destiné à déterminer le volume de fluide aspiré hors de l'intérieur du premier corps creux.

13. Procédé pour la fabrication d'un dispositif (10, 20) selon l'une quelconque des revendications 1 à 11, comprenant les étapes suivantes:
(i) préparer au moins un premier corps creux (1) avec une structure de paroi souple et pouvant être rempli avec un premier fluide,
(ii) préparer un deuxième corps creux (2) avec une structure d'enveloppe extérieure souple, dans lequel la structure d'enveloppe extérieure présente au moins une première ouverture (3), qui peut simuler une plaie,
et dans lequel ledit au moins un premier corps creux (1) peut être introduit à l'intérieur du deuxième corps creux (2),
(iii) remplir ledit au moins un premier corps creux (1) rempli avec un premier fluide avec un fluide, en particulier avec de l'eau ou de l'air,
(iv) fermer la multiplicité de deuxièmes corps creux (2), de telle manière qu'aucun fluide ne puisse s'échapper de l'intérieur des corps creux,
(v) introduire ledit au moins un premier corps creux (1) rempli avec un fluide à l'intérieur du deuxième corps creux (2), de telle manière que l'intérieur du deuxième corps creux (2) soit très largement rempli.

14. Procédé pour la mesure de la capacité de drainage d'un pansement prévu pour la thérapie en dépression de plaies, comprenant les étapes suivantes:
(i) préparer un dispositif de mesure (30) selon la revendication 12,
(ii) établir une liaison de fluide (32) entre une source de fluide (31) et l'intérieur du deuxième corps creux (2),
dans lequel soit le moyen de conduite (32) est mené à travers la deuxième ouverture (4) jusqu'à l'intérieur du premier corps creux (1),
soit
le moyen de conduite est raccordé à une pièce de raccord, qui est prévue à la deuxième ouverture (4),
(iii) appliquer un système de thérapie en dépression (33; 34; 35; 36; 37; 38; 39), dans lequel le pansement de thérapie en dépression (36; 37; 38; 39) est posé sur la première ouverture (3),
(iv) mettre en service le système de thérapie en dépression et introduire un volume de fluide désiré à l'intérieur du deuxième corps creux (2),
(v) déterminer le volume de fluide aspiré hors de l'intérieur du deuxième corps creux (2) par unité de temps,
(vi) en option, saisir la pression intérieure Pᵢ, la température intérieure Tᵢ et éventuellement d'autres données de mesure,
(vii) en option, saisir et traiter les données de mesure au moyen d'un ordinateur.
